# FASCICULE DE BREVET EUROPEEN

(11) **EP 3 798 651 B1**
(45) Date de publication et mention de la délivrance du brevet: **14.06.2023**
(21) Numéro de dépôt: 20186414.7
(22) Date de dépôt: 17.07.2020
(51) Int. Cl.: G01R 31/12

(54) **SYSTÈME DE SURVEILLANCE DES DÉCHARGES PARTIELLES DANS UN APPAREILLAGE ÉLECTRIQUE VIA LES ÉMISSIONS GAZEUSES**
SYSTEM ZUR ÜBERWACHUNG DER TEILENTLADUNGEN IN EINEM ELEKTRISCHEN GERÄT ÜBER DIE GASEMISSIONEN
SYSTEM FOR MONITORING PARTIAL DISCHARGES IN ELECTRICAL APPARATUS VIA GAS EMISSIONS

(30) Priorité: 25.09.2019 FR 1910558
(43) Date de publication de la demande: 31.03.2021
(73) Titulaire: Schneider Electric Industries SAS, 92500 Rueil Malmaison (FR)
(72) Inventeur: FERRARO, Venanzio, 38000 Grenoble (FR); ALBERTO, Diego, 38700 Corenc (FR); DURAND, Maxime, 38000 Grenoble (FR)
(74) Mandataire: Schneider Electric

(56) Documents cités:
- EP-A1- 3 258 278
- CN-A- 105 606 666
- US-A- 5 107 447
- US-A1- 2009 119 035
- US-A1- 2016 356 852

## Description

### Domaine technique

La présente invention concerne un système et un procédé de surveillance des décharges partielles dans au moins un appareillage électrique de moyenne tension ou haute tension.

L'invention concerne également une installation électrique comportant un tel système de surveillance.

### Technique antérieure

Les appareillages électriques de moyenne et haute tension sont constamment sollicités par des hauts niveaux de courant et de tension électriques, et peuvent être soumis à des décharges partielles.

Dans ce qui suit, les termes « moyenne tension » et « haute tension » sont utilisés dans leur acceptation habituelle, à savoir que le terme « moyenne tension » désigne une tension qui est supérieure à 1 000 volts en courant alternatif et à 1 500 volts en courant continu mais qui ne dépasse pas 52 000 volts en courant alternatif et 75 000 volts en courant continu, tandis que le terme « haute tension » désigne une tension qui est strictement supérieure à 52 000 volts en courant alternatif et à 75 000 volts en courant continu.

Une décharge partielle est une décharge électrique localisée qui court-circuite partiellement un matériau isolant séparant des matériaux conducteurs, sous l'effet d'une forte tension.

Les décharges partielles peuvent notamment être causées par un défaut de fabrication, à des conditions environnementales non maitrisé ou à une usure des matériaux.

A titre d'exemple, les décharges partielles dans un matériau diélectrique peuvent avoir lieu dans une bulle gazeuse : la permittivité du gaz étant très inférieure à celle du matériau environnant, un champ électrique significativement plus important que celui existant au sein du matériau isolant apparait. Si la tension électrique dépasse la limite de rigidité diélectrique pour le gaz contenu par la bulle, une décharge partielle a lieu. À l'inverse, la présence d'une particule conductrice dans l'isolant peut également mener à une décharge partielle.

D'autres types de décharges partielles peuvent avoir lieu en surface du matériau isolant ou dans les gaz environnants par effet corona.

On distingue ainsi deux types de décharges partielles, à savoir les décharges partielles internes, se produisant à l'intérieur d'un matériau isolant, et les décharges partielles externes, se produisant à la surface ou dans les gaz environnant directement le matériau isolant.

Ces décharges partielles sont susceptibles de dégrader de façon accélérée la tenue diélectrique du matériau isolant, par exemple en favorisant une érosion ou une oxydation du matériau. Sur le moyen terme et/ou long terme, ces dégradations peuvent devenir significatives et conduire finalement à un claquage aux bornes du matériau isolant et à la destruction de l'appareillage électrique.

Il en résulte un risque accru pour la sécurité de l'appareillage électrique et donc des personnes qui travaillent à proximité.

Afin de limiter ce risque, on connait différents procédés de surveillance des décharges partielles dans un appareillage électrique, par exemple des méthodes électriques, acoustiques ou optiques.

Le document EP 3 258 278 A1 présente ainsi un procédé basé sur une analyse acoustique corrélée avec des mesures de capteurs de température et d'humidité.

Le document EP 1 593 981 A2 présente un procédé basé sur une analyse des signaux électriques de tension et de courant lorsqu'une décharge partielle apparait. Le document US 5,107,447 A présente également une méthode basée sur une analyse des signaux électriques de tension et de courant lorsqu'une décharge partielle se produit, cependant son appareil de détection d'anomalie détecte en outre une anomalie d'alimentation sur la base des mesures de température.

Des procédés chimiques peuvent également être utilisés. En effet, des décharges partielles externes peuvent entrainer des réactions chimiques à la surface des matériaux isolants de l'appareillage électrique ou dans le gaz environnant, notamment l'air. Ces réactions chimiques entrainent la formation d'éléments gazeux, notamment d'ozone.

Le document « Characterization of medium voltage equipment ageing by monitoring of partial discharges chemical and acoustical émission, E. Odic, E. Jouseau, G. Vivien, C-S. Maroni, 10th International Electrical Insulation Conf. INSUCON 2006, Birmingham: United Kingdom (2006) » présente ainsi un procédé de surveillance des décharges partielles utilisant des capteurs d'ozone.

Toutefois, un tel procédé ne permet pas de déterminer la cause spécifique des décharges partielles. Or, les actions à entreprendre pour limiter l'apparition de ces décharges partielles peuvent en dépendre.

En outre, un tel procédé peut se révéler imprécis dans certaines situations. En effet, la concentration des éléments gazeux mesurée par les capteurs est susceptible de varier au cours du temps indépendamment de l'apparition ou non de décharges partielles, par exemple en fonction d'autres activités humaines réalisées à proximité de l'appareillage électrique.

A titre d'exemple, il est connu que certaines usines ou que le trafic automobile peuvent émettre des précurseurs d'ozone, tels que l'oxyde d'azote, des hydrocarbures, ou des composés organiques volatiles. Ces précurseurs d'ozone peuvent ensuite réagir, notamment sous l'action des radiations ultraviolettes du soleil, en ozone.

Ainsi, les mesures des capteurs d'éléments gazeux sont susceptibles d'être faussées en mesurant des concentrations élevées d'éléments gazeux, tels que l'ozone, indépendamment de l'apparition ou non de décharges partielles dans l'appareillage électrique.

Un des buts de l'invention est donc de trouver un système simple, fiable et économique qui permet de détecter la survenance de décharges partielles dans une installation électrique, d'une part afin d'en connaitre la cause et d'autre part de s'assurer de l'efficacité du procédé utilisé.

En outre, les éléments gazeux produits lors de décharges partielles peuvent avoir un impact significatif sur la santé, en favorisant par exemple une réaction inflammatoire des bronches ou des lésions du tissu pulmonaire chez les personnes travaillant à proximité de l'appareillage électrique.

Un autre but de l'invention est donc de permettre une surveillance continue et en temps réel permettant de détecter des décharges partielles potentiellement dangereuses.

### Résumé

L'invention vient améliorer la situation.

Il est proposé un procédé de surveillance de décharges partielles dans une installation électrique comprenant au moins une cellule électrique, la cellule électrique comprenant au moins un appareillage électrique de moyenne tension ou haute tension, l'installation électrique comportant :
- au moins un premier ensemble de capteurs disposé à l'intérieur de la cellule électrique et au moins un second ensemble de capteurs disposé à l'extérieur de la cellule électrique, chacun des premier et second ensembles de capteurs comprenant au moins un capteur d'un élément gazeux,
- au moins un capteur de température et/ou un capteur d'humidité disposés à l'intérieur de l'installation électrique,
le procédé comprenant au moins les étapes suivantes :
a1) comparer les concentrations de l'élément gazeux obtenue avec le premier ensemble de capteur et le second ensemble de capteurs,
a2) déterminer à partir de la comparaison de l'étape a1) si une variation de la concentration d'élément gazeux acquise par le premier ensemble de capteurs provient d'un facteur extérieur à l'installation électrique,
b1) déterminer si une corrélation existe entre la concentration de l'élément gazeux obtenue par le premier ensemble de capteur et les mesures du capteur de température et/ou d'humidité,
b2) déterminer à partir de l'étape b1) si une variation de la concentration de l'élément gazeux acquis par le premier ensemble de capteurs provient d'une variation de température et/ou d'humidité à l'intérieur de l'installation électrique, et
c) si la réponse aux étapes a2) et b2) est négative, déduire que la concentration de l'élément gazeux obtenue par le premier ensemble de capteurs provient d'un facteur intérieur à l'installation électrique.

Grâce à ces dispositions, il est possible de distinguer entre les décharges partielles provenant de facteurs extérieures, tels que conditions environnementales particulières à l'intérieur de l'installation électrique, qui peuvent favoriser l'apparition de décharges partielles de façon ponctuelle, et les décharges partielles provenant plus particulièrement de facteurs intérieurs à l'appareillage électrique, tels que l'usure des matériaux de l'appareillage électriques.

Les caractéristiques exposées dans les paragraphes suivants peuvent, optionnellement, être mises en oeuvre. Elles peuvent être mises en oeuvre indépendamment les unes des autres ou en combinaison les unes avec les autres :

Selon une réalisation, le capteur d'un élément gazeux des premier et second ensembles de capteurs est choisi parmi un capteur d'ozone, un capteur de monoxyde de carbone, un capteur d'acide nitrique et un capteur d'oxydes d'azote.

Selon une autre réalisation, si la réponse à l'étape b2) est positive, le procédé comprend en outre une étape b3) de régulation des conditions environnementales à l'intérieur de l'installation électrique.

Selon une autre réalisation, si la réponse à l'étape b2) est négative, le procédé comprend en outre une étape c') de détermination de l'état d'usure du matériau de l'appareillage électrique en fonction de la concentration de l'élément gazeux obtenue par le premier ensemble de capteur.

Selon une autre réalisation, le procédé comprend en outre une étape d) d'alerte si la concentration de l'élément gazeux obtenue par le premier ensemble de capteur est supérieure à une valeur seuil.

Selon une autre réalisation, le facteur intérieur est une usure de l'appareillage électrique.

Une analyse « différentielle » ou relative entre les capteurs installés à l'intérieur et à l'extérieur de la cellule électrique permet donc de garantir une maintenance satisfaisante de l'installation électrique. Une analyse des concentrations absolues permet de surveiller les concentrations d'éléments gazeux et de garantir ainsi si nécessaire la sécurité des personnes travaillant dans l'installation électrique.

Selon un autre aspect, il est proposé un système de surveillance de décharges partielles dans une installation électrique comprenant au moins une cellule électrique, la cellule électrique comprenant au moins un appareillage électrique de moyenne tension ou haute tension, le système comportant :
- au moins un premier ensemble de capteurs disposé à l'intérieur de la cellule électrique et au moins un second ensemble de capteurs disposé à l'extérieur de la cellule électrique, chacun des premier et second ensembles de capteurs comprenant au moins un capteur d'un élément gazeux,
- au moins un capteur de température et/ou un capteur d'humidité disposés à l'intérieur de l'installation électrique,
- une unité de traitement comprenant un processeur configuré pour mettre en oeuvre au moins les étapes suivantes :
   a1) comparer les concentrations de l'élément gazeux obtenues avec le premier ensemble de capteur et le second ensemble de capteurs,
   a2) déterminer à partir de la comparaison de l'étape a1) si une variation de la concentration d'élément gazeux acquise par le premier ensemble de capteurs provient d'un facteur extérieur à l'installation électrique,
   b1) déterminer si une corrélation existe entre la concentration de l'élément gazeux obtenue par le premier ensemble de capteur et les mesures du capteur de température et/ou d'humidité,
   b2) déterminer à partir de l'étape b1) si une variation de la concentration de l'élément gazeux acquis par le premier ensemble de capteurs provient d'une variation de la température ou de l'humidité à l'intérieur de l'installation électrique, et
   c) si la réponse aux étapes a2) et b2) est négative, déduire que la concentration de l'élément gazeux obtenue par le premier ensemble de capteurs provient d'un facteur intérieur à l'installation électrique.

Selon une réalisation, le système comprend un dispositif d'alarme.

Selon une autre réalisation, le système comprend un ou plusieurs actionneurs configurés pour réguler les conditions environnementales à l'intérieur de l'installation électrique.

### Brève description des dessins

D'autres caractéristiques, détails et avantages apparaîtront à la lecture de la description détaillée ci-après, et à l'analyse des dessins annexés, sur lesquels :
**Fig. 1**

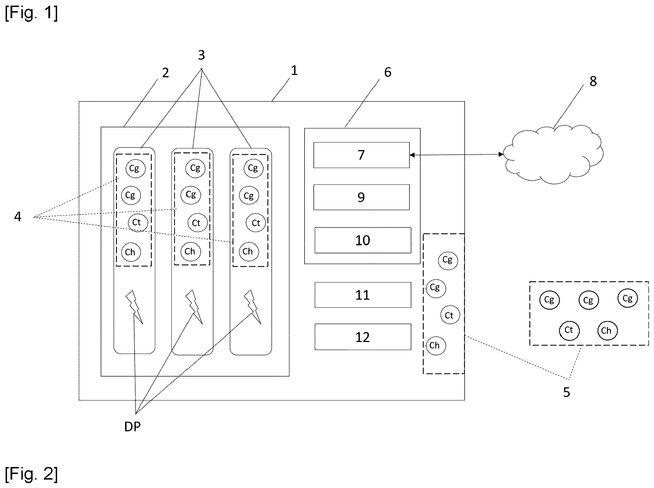

   [Fig. 1] représente une vue schématique d'une installation selon un mode de réalisation de l'invention.
**Fig. 2**

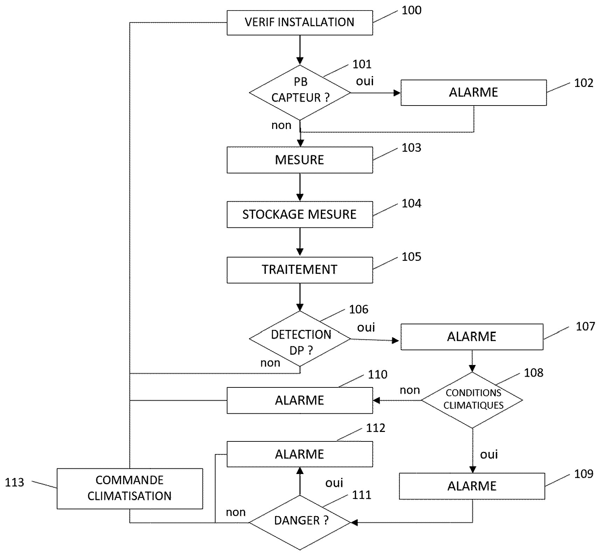

   [Fig. 2] représente une vue schématique d'un procédé de surveillance selon un mode de réalisation de l'invention.

### Description des modes de réalisation

Les dessins et la description ci-après contiennent, pour l'essentiel, des éléments de caractère certain. Ils pourront donc non seulement servir à mieux faire comprendre la présente divulgation, mais aussi contribuer à sa définition, le cas échéant.

La [Fig. 1] illustre de façon schématique une installation ou poste électrique 1 comprenant un ou plusieurs appareillages électriques 3 disposés dans une cellule électrique 2.

Selon le mode de réalisation illustré sur la [Fig. 1], l'installation électrique 1 comprend trois appareillages électriques 3 disposés dans une même cellule électrique 2.

L'installation électrique 1 peut être une station d'un réseau électrique servant à la transmission et/ou à la distribution d'électricité. L'appareillage électrique 3 peut être choisi parmi un interrupteur, un disjoncteur, un contacteur, un interrupteur fusible, un recloser ou un sectionneur. Toutefois, d'autres types d'appareillages électriques 3 sont possibles.

De façon connu en soi, l'appareillage électrique 3 comprend plusieurs matériaux métalliques (tels qu'acier, cuivre ou aluminium) ou isolants (tels qu'élastomère, résine ou éthylène-propylène-diène monomère). Toutefois, l'utilisation d'autre types de matériaux est bien entendu possible, les contraintes de nature diélectrique des matériaux étant plus particulièrement choisis en fonction de l'application de l'appareillage électrique visée.

L'installation électrique 1 comprend un système de surveillance des décharges partielles DP, notamment externes. Le système de surveillance comprend au moins un premier ensemble de capteurs 4 (appelé par la suite « premier ensemble ») et un second ensemble de capteurs 5 (appelé par la suite « second ensemble »).

Le premier ensemble 4 est disposé à l'intérieur de la cellule électrique 2, notamment à proximité de l'appareillage électrique 3, tandis que le second ensemble 5 est disposé à l'extérieur de la cellule électrique 2, notamment à l'intérieur et/ou à l'extérieur de l'installation électrique 1.

Selon le mode de réalisation illustré sur la [Fig. 1], le premier ensemble 4 comprend des capteurs disposés au niveau de chacun des appareillages électriques 3 et le second ensemble 5 comprend des capteurs disposés à la fois à l'intérieur et à l'extérieur de l'installation électrique1.

Le premier ensemble 4 comprend au moins un capteur d'éléments gazeux Cg.

Le capteur d'éléments gazeux Cg est configuré pour mesurer une variation ou une concentration d'un élément gazeux. L'élément gazeux peut être choisi parmi l'ozone (O3), le monoxyde de carbone (CO), l'acide nitrique (HNO3) et les oxydes d'azote (Nox). Avantageusement, l'élément gazeux est l'ozone, qui est l'un des premiers gaz générés lors d'une décharge partielle externes. Toutefois, d'autres éléments gazeux sont également envisageables.

Le premier ensemble 4 comprend également au moins un capteur de température Ct et/ou un capteur d'humidité Ch. Avantageusement, le premier ensemble 4 comprend à la fois un capteur de température Ct et un capteur d'humidité Ch.

Le premier ensemble 4 peut également comprendre un ou plusieurs autres capteurs additionnels configurés pour acquérir une mesure environnementale, telle qu'une mesure de pression, d'altitude, ou autre. Ces capteurs additionnels peuvent permettre de prendre en compte des conditions climatiques spécifiques à l'installation électrique 1 lors de l'analyse des mesures acquises, fonction de sa position géographique par exemple.

Les capteurs du premier ensemble 4 peuvent être disposés dans la cellule électrique 2, notamment à proximité immédiate de l'appareillage électrique 3. Les capteurs du premier ensemble 4 peuvent par exemple être disposés en contact, notamment sur la paroi extérieure, de l'appareillage électrique 3 ou à distance immédiate de l'appareillage électrique 3 pour mesurer une variation ou une concentration d'un élément gazeux situé dans l'air autour de l'appareillage électrique 3.

Le second ensemble 5 comprend au moins un capteur d'élément gazeux Cg. De la même façon que pour le premier ensemble 4, le capteur d'éléments gazeux Cg est configuré pour mesurer une variation ou une concentration d'un élément gazeux. L'élément gazeux peut être choisi parmi l'ozone (O3), le monoxyde de carbone (CO), l'acide nitrique (HNO3) et l'oxydes d'azote (Nox). Le second ensemble 5 comprend ainsi avantageusement les mêmes capteurs d'élément gazeux que le premier ensemble 4.

Le second ensemble 5, situé à l'extérieur la cellule électrique 2, permet de mesurer les variations ou la concentration des éléments gazeux au cours du temps, indépendamment du fonctionnement de l'appareillage électrique 3 et de l'apparition de décharges partielles.

En effet, comme indiqué précédemment, les concentrations des certains éléments gazeux sont susceptibles de varier journalièrement ou saisonnièrement, par exemple en fonction d'activités humaines réalisées dans un environnement proche de l'installation électrique 1. Le second ensemble 5 permet de prendre en compte ces variations.

De la même façon que pour le premier ensemble 4, le second ensemble 5 peut comprendre également au moins un capteur de température Ct et/ou un capteur d'humidité Ch. Avantageusement, le second ensemble 5 comprend à la fois un capteur de température Ct et un capteur d'humidité Ch.

Le système comprend également une unité de traitement 6. L'unité de traitement 6 est configurée pour analyser et traiter les mesures une fois acquises par les différents capteurs des premier et second ensemble 4, 5.

L'unité de traitement 6 comprend ainsi une interface de communication 7 configurée pour recevoir les mesures acquises par les capteurs. La communication entre l'unité de traitement 6 et les capteurs peut être réalisée de façon filaire ou sans fil, notamment via des protocoles courtes distances tels que Bluetooth, Sigfox, LoRa, ou autre.

L'interface de communication 7 permet également d'émettre/de recevoir des informations provenant d'un serveur distant 8. Le serveur distant 8 peut ainsi être configuré pour analyser et traiter les mesures acquises par les capteurs à distance en combinaison ou en variante de l'unité de traitement 6. La communication entre l'unité de traitement 6 et le serveur distant 8 peut être réalisées de façon filaire ou sans fil, notamment via des protocoles longues distances tels qu'Ethernet ou 3G/4G/5G.

L'unité de traitement 6 comprend également un processeur 9 configuré pour analyser, avantageusement en temps réel, les mesures acquises par les capteurs. Le processeur 9 peut mettre en oeuvre des outils mathématiques ou algorithmiques, notamment en utilisant une intelligence artificielle.

Par en temps réel, on entend que le procédé selon l'invention est mis en oeuvre pendant le fonctionnement de l'installation électrique 1, dans des limites temporelles fixées.

L'unité de traitement 6 comprend en outre une mémoire 10 configurée pour stocker les mesures acquises au cours du temps par les capteurs.

Le système comprend également un ou plusieurs dispositifs d'alarme 11, notamment sonore, lumineux, par message ou autres, configurés pour avertir les personnes travaillant pour l'installation électrique 1, notamment en cas de concentrations d'éléments gazeux élevées comme cela sera décrit ci-après.

Le système comprend également un ou plusieurs actionneurs 12. Un actionneur 12 est configuré pour contrôler et/ou réguler les conditions environnementales à l'intérieur de l'installation électrique 1, et plus particulièrement à l'intérieur de la cellule électrique 2. Un actionneur 12 peut par exemple commander un dispositif de chauffage, de refroidissement, d'humidification ou de déshumidification à l'intérieur de l'installation électrique 1.

On décrit ci-après, et en lien avec la [Fig. 2], le procédé de surveillance de décharges partielles à l'intérieur de l'installation électrique 1 selon l'invention.

Dans une étape 100, le système vérifie l'intégrité et le bon fonctionnement de l'installation électrique 1, et notamment du système de surveillance. En particulier, dans une étape 101, le système vérifie le bon fonctionnement des premier et second ensembles 4, 5.

Si un capteur ne fonctionne pas ou a un problème de communication avec l'unité de traitement 6, le système envoie un message d'alarme dans une étape 102 via le dispositif d'alarme 11.

Si aucun problème n'est détecté, le système procède dans une étape 103 à l'acquisition des mesures des capteurs. Ces mesures peuvent être stockées dans la mémoire 10 du système de traitement 6 et/ou sur le serveur distant 8 (étape 104).

Dans une étape 105, le système compare les mesures acquises entre le premier ensemble 4 et le second ensemble de capteurs 5.

Comme évoqué précédemment, certains éléments gazeux, tels que l'ozone, peuvent être produits par des décharges partielles mais également par d'autres facteurs extérieurs et indépendants de l'installation électrique 1. Il est donc nécessaire de comparer les concentrations des éléments gazeux obtenues par le premier ensemble 4, situé dans la cellule électrique 2, avec les concentrations des éléments gazeux obtenues par le second ensemble 5, situé hors de la cellule électrique 2. Cette comparaison peut être réalisée par le processeur 9 de l'unité de traitement 6 et/ou de façon déportée sur le serveur distant 8 (étapes 105 et 106).

Par « facteur extérieur », on comprend une cause partielle ou totale de la concentration des éléments gazeux, générée ou non par l'apparition de décharges partielles, qui n'est pas liée à l'état ou au fonctionnement de l'appareillage électrique 3. Un facteur extérieur est donc par exemple des conditions environnementales et climatiques impactant l'intérieur de l'installation électrique 1 ou une émission d'éléments gazeux par des éléments indépendant de l'installation électrique 1.

Par opposition à un facteur extérieur, on comprend par « facteur intérieur » une cause partielle ou totale de la concentration des éléments gazeux qui est liée à l'état ou au fonctionnement de l'appareillage électrique 3. Un facteur interne est par exemple une usure ou un défaut de fabrication de l'appareillage électrique 3.

Si la comparaison indique une différence inférieure à une valeur seuil donnée, et/ou une corrélation en termes de concentration et de temporalité entre les mesures acquises par les premier et second ensembles 4, 5, alors il est possible de conclure que les concentrations d'éléments gazeux mesurées proviennent de facteurs extérieurs à l'installation électriques 1. Elles ne sont donc pas liées à des décharges partielles apparues au sein de la cellule électrique 2.

Si la comparaison indique une différence supérieure à une valeur seuil donnée, et/ou une absence de corrélation en termes de concentration et de temporalité entre les mesures acquises par les premier et second ensembles 4, 5, alors il est possible de conclure que les concentrations d'éléments gazeux proviennent de décharges partielles apparues au sein de la cellule électrique 2.

Le système envoie alors un message d'alarme dans une étape 107 via le dispositif d'alarme 11.

Ces décharges partielles peuvent notamment être causées par un facteur intérieur, notamment une usure des matériaux isolants de l'appareillage électrique 3.

Selon un mode de réalisation non illustré, le système peut mesurer la dégradation, notamment par oxydation, des matériaux de l'appareillage électrique 3 en fonction des concentrations d'éléments gazeux acquises par les capteurs. On peut ainsi estimer la dégradation des appareillages électriques 3 en prenant en compte des modèles connus d'usure et de vieillissement des matériaux.

En particulier l'ordre d'apparition et la concentration relatives des différents éléments gazeux permet de connaitre la cinétique des réactions chimiques faisant suite à une décharge partielle, et d'affiner l'estimation de la dégradation du matériau de l'appareillage électrique 3 considéré.

Cependant, ces décharges partielles peuvent également être favorisées par des facteurs extérieurs, tels que certaines conditions environnementales particulières à l'intérieur de l'installation électrique 1, notamment de température et d'humidité à l'intérieur de la cellule électrique 2. En particulier, l'amplitude ou la fréquence de décharges partielles sont susceptibles d'augmenter ou de diminuer si la température et/ou l'humidité varie.

Si les capteurs montrent une corrélation entre les conditions environnementales (en particulier température ou humidité) à l'intérieur de l'installation électrique 1 et les concentrations des éléments gazeux (étape 108), il est possible de conclure que les décharges partielles sont liées aux conditions environnementales à l'intérieur de l'installation électrique 1. Le système envoie un message d'alarme dans une étape 109 via le dispositif d'alarme 11.

A titre d'exemple, les concentrations des éléments gazeux peuvent être comparées à des concentrations seuil connus de ces mêmes éléments gazeux normalement observées pour une même température ou humidité à l'intérieur de l'installation électrique 1.

Si les capteurs montrent une corrélation insuffisante, il est possible de conclure que les décharges partielles sont plutôt liées à un facteur intérieur, tel qu'une usure des matériaux de l'appareillage électrique 3. Il est par conséquent nécessaire de prévoir une maintenance de l'appareillage électrique afin de garantir la sécurité et le bon fonctionnement de l'installation.

En outre, dans une étape 111, le système évalue si les concentrations des élément gazeux à l'intérieur de l'installation électrique 1 sont susceptibles d'avoir un impact négatif sur la santé des personnes travaillant à l'intérieur de l'installation électrique 1. En cas de concentrations supérieures à des valeurs seuils données, le système envoie alors un message d'alarme dans une étape 112 via le dispositif d'alarme 11.

Selon le mode de réalisation illustré sur la [Fig. 1] dans lequel l'installation électrique 1 comprend des actionneurs 12, le système peut permettre, dans une étape 113, de contrôler et/ou de réguler les conditions environnementales à l'intérieur de l'installation électrique 1 afin de limiter les risques liés aux décharges partielles. Le système est ainsi capable d'adapter la température et l'humidité dans l'installation électrique 1 afin de réduire l'apparition de décharges partielles, et par conséquent la génération des éléments gazeux.

Le procédé peut être mis en oeuvre au moyen des capteurs du premier ensemble 4 disposés à proximité de chacun des appareillages électriques 3. La disposition de ces capteurs permet ainsi de localiser plus précisément quel appareillage électrique 3 ou cellule électrique 2 nécessite d'être plus particulièrement surveillé.

Le procédé selon l'invention permet d'améliorer la détection des décharges partielles, d'en déterminer les causes (notamment usure des matériaux ou conditions environnementales à l'intérieur de la cellule électrique), puis d'entreprendre des actions à l'intérieur de l'installation électrique 1 afin de limiter l'apparition de nouvelles décharges partielles ou de prévenir les personnes concernées au moyen de différents niveaux d'alarmes.

Selon le mode de réalisation illustré sur la [Fig. 2], le procédé peut ensuite être répété en reprenant toutes ou partie des étapes 100 à 113 décrites ci-dessus. Le procédé est ainsi mis en oeuvre de façon continue.

Par « de façon continue », on entend que le procédé est mis en oeuvre de façon répétée avantageusement pendant toute la durée de fonctionnement de l'installation électrique 1.

L'invention est définie par les revendications annexées.

## Revendications

1. Procédé de surveillance de décharges partielles, DP, dans une installation électrique (1) comprenant au moins une cellule électrique (2), la cellule électrique (2) comprenant au moins un appareillage électrique (3) de moyenne tension ou haute tension, l'installation électrique (1) comportant :
- au moins un premier ensemble de capteurs (4) disposé à l'intérieur de la cellule électrique (2) et au moins un second ensemble de capteurs (5) disposé à l'extérieur de la cellule électrique (2), chacun des premier et second ensembles de capteurs (4, 5) comprenant au moins un capteur d'un élément gazeux (Cg),
- au moins un capteur de température (Ct) et/ou un capteur d'humidité (Ch) disposés à l'intérieur de l'installation électrique (1),
**caractérisé en ce que** le procédé comprend au moins les étapes suivantes :
a1) comparer les concentrations de l'élément gazeux obtenues avec le premier ensemble de capteurs (4) et le second ensemble de capteurs (5), a2) déterminer à partir de la comparaison de l'étape a1) si une variation de la concentration d'élément gazeux acquise par le premier ensemble de capteurs (4) provient d'un facteur extérieur à l'installation électrique (1),
b1) déterminer si une corrélation existe entre la concentration de l'élément gazeux obtenue par le premier ensemble de capteur (4) et les mesures du capteur de température (Ct) et/ou d'humidité (Ch),
b2) déterminer à partir de l'étape b1) si une variation de la concentration de l'élément gazeux acquis par le premier ensemble de capteurs (4) provient d'une variation de température et/ou d'humidité à l'intérieur de l'installation électrique (1), et
c) si la réponse aux étapes a2) et b2) est négative, déduire que la concentration de l'élément gazeux obtenue par le premier ensemble de capteurs (4) provient d'un facteur intérieur à l'installation électrique (1).

2. Procédé selon la revendication 1, dans lequel le capteur d'un élément gazeux des premier et second ensembles de capteurs (4, 5) est choisi parmi un capteur d'ozone (O3), un capteur de monoxyde de carbone (CO), un capteur d'acide nitrique (HNO3) et un capteur d'oxydes d'azote (Nox).

3. Procédé selon l'une quelconque des revendications précédentes, dans lequel si la réponse à l'étape b2) est positive, le procédé comprend en outre une étape b3) de régulation des conditions environnementales à l'intérieur de l'installation électrique (1).

4. Procédé selon la revendication 1 ou 2, dans lequel si la réponse à l'étape b2) est négative, le procédé comprend en outre une étape c') de détermination de l'état d'usure du matériau de l'appareillage électrique (3) en fonction de la concentration de l'élément gazeux obtenue par le premier ensemble de capteur (4).

5. Procédé selon l'une quelconque des revendications précédentes, comprenant en outre une étape d) d'alerte si la concentration de l'élément gazeux obtenue par le premier ensemble de capteur (4) est supérieure à une valeur seuil.

6. Procédé selon l'une quelconque des revendications précédentes, dans lequel le facteur intérieur est une usure de l'appareillage électrique (3).

7. Système de surveillance de décharges partielles, DP, dans une installation électrique (1) comprenant au moins une cellule électrique (2), la cellule électrique (2) comprenant au moins un appareillage électrique (3) de moyenne tension ou haute tension, le système comportant :
- au moins un premier ensemble de capteurs (4) disposé à l'intérieur de la cellule électrique (2) et au moins un second ensemble de capteurs (5) disposé à l'extérieur de la cellule électrique (2), chacun des premier et second ensemble de capteurs (4, 5) comprenant au moins un capteur d'un élément gazeux (Cg),
- au moins un capteur de température (Ct) et/ou un capteur d'humidité (Ch) disposés à l'intérieur de l'installation électrique (2),
- une unité de traitement (6) comprenant un processeur (9) configuré pour mettre en oeuvre au moins les étapes suivantes :
a1) comparer les concentrations de l'élément gazeux obtenue avec le premier ensemble de capteur (4) et le second ensemble de capteurs (5),
a2) déterminer à partir de la comparaison de l'étape a1) si une variation de la concentration d'élément gazeux acquise par le premier ensemble de capteurs (4) provient d'un facteur extérieur à l'installation électrique (1),
b1) déterminer si une corrélation existe entre la concentration de l'élément gazeux obtenue par le premier ensemble de capteur (4) et les mesures du capteur de température (Ct) et/ou d'humidité (Ch),
b2) déterminer à partir de l'étape b1) si une variation de la concentration de l'élément gazeux acquis par le premier ensemble de capteurs (4) provient d'une variation de la température ou de l'humidité à l'intérieur de l'installation électrique (1), et
c) si la réponse aux étapes a2) et b2) est négative, déduire que la concentration de l'élément gazeux obtenue par le premier ensemble de capteurs (4) provient d'un facteur intérieur à l'installation électrique (1).

8. Système selon la revendication 7, comprenant un dispositif d'alarme (11).

9. Système selon la revendication 7 ou 8, comprenant un ou plusieurs actionneurs (12) configurés pour réguler les conditions environnementales à l'intérieur de l'installation électrique (1).

## Patentansprüche

1. Verfahren zur Überwachung von Teilentladungen, DP, in einer elektrischen Anlage (1), die mindestens eine elektrische Zelle (2) umfasst, wobei die elektrische Zelle (2) mindestens ein elektrisches Mittelspannungs- oder Hochspannungsgerät (3) umfasst, wobei die elektrische Anlage (1) beinhaltet:
- mindestens eine erste Sensorenanordnung (4), die innerhalb der elektrischen Zelle (2) angeordnet ist, und mindestens eine zweite Sensorenanordnung (5), die außerhalb der elektrischen Zelle (2) angeordnet ist, wobei jede der ersten und zweiten Sensorenanordnungen (4, 5) mindestens einen Sensor für ein gasförmiges Element (Cg) umfasst,
- mindestens einen Temperatursensor (Ct) und/oder einen Feuchtesensor (Ch), die innerhalb der elektrischen Anlage (1) angeordnet sind, **dadurch gekennzeichnet, dass** das Verfahren mindestens die folgenden Schritte umfasst:
a1) Vergleichen der Konzentrationen des gasförmigen Elements, die mit der ersten Sensorenanordnung (4) und der zweiten Sensorenanordnung (5) erhalten wurden,
a2) Bestimmen, ausgehend von dem Vergleich des Schritts a1), ob eine Änderung der von der ersten Sensorenanordnung (4) erfassten Konzentration des gasförmigen Elements auf einem außerhalb der elektrischen Anlage (1) liegenden Faktor beruht,
b1) Bestimmen, ob eine Korrelation zwischen der von der ersten Sensorenanordnung (4) erhaltenen Konzentration des gasförmigen Elements und den Messungen des Temperatursensors (Ct) und/oder Feuchtesensors (Ch) vorliegt,
b2) Bestimmen, ausgehend vom Schritt b1), ob eine Änderung der von der ersten Sensorenanordnung (4) erfassten Konzentration des gasförmigen Elements auf einer Temperatur- und/oder Feuchteänderung innerhalb der elektrischen Anlage (1) beruht, und
c) wenn die Antwort in den Schritten a2) und b2) negativ ist, Schlussfolgern, dass die von der ersten Sensorenanordnung (4) erhaltene Konzentration des gasförmigen Elements auf einem innerhalb der elektrischen Anlage (1) liegenden Faktor beruht.

2. Verfahren nach Anspruch 1, bei dem der Sensor für ein gasförmiges Element der ersten und zweiten Sensorenanordnungen (4, 5) unter einem Ozonsensor (O3), einem Kohlenstoffmonoxidsensor (CO), einem Salpetersäuresensor (HNO3) und einem Stickoxidsensor (Nox) gewählt ist.

3. Verfahren nach einem der vorhergehenden Ansprüche, bei dem das Verfahren, wenn die Antwort im Schritt b2) positiv ist, ferner einen Schritt b3) des Regulierens der Umgebungsbedingungen innerhalb der elektrischen Anlage (1) umfasst.

4. Verfahren nach Anspruch 1 oder 2, bei dem das Verfahren, wenn die Antwort im Schritt b2) negativ ist, ferner einen Schritt c') des Bestimmens des Verschleißzustands des Materials des elektrischen Geräts (3) in Abhängigkeit von der von der ersten Sensorenanordnung (4) erhaltenen Konzentration des gasförmigen Elements umfasst.

5. Verfahren nach einem der vorhergehenden Ansprüche, umfassend ferner einen Schritt d) des Warnens, wenn die von der ersten Sensorenanordnung (4) erhaltene Konzentration des gasförmigen Elements über einem Schwellenwert liegt.

6. Verfahren nach einem der vorhergehenden Ansprüche, bei dem der innerhalb liegende Faktor ein Verschleiß des elektrischen Geräts (3) ist.

7. System zur Überwachung von Teilentladungen, DP, in einer elektrischen Anlage (1), die mindestens eine elektrische Zelle (2) umfasst, wobei die elektrische Zelle (2) mindestens ein elektrisches Mittelspannungs- oder Hochspannungsgerät (3) umfasst, wobei das System beinhaltet:
- mindestens eine erste Sensorenanordnung (4), die innerhalb der elektrischen Zelle (2) angeordnet ist, und mindestens eine zweite Sensorenanordnung (5), die außerhalb der elektrischen Zelle (2) angeordnet ist, wobei jede der ersten und zweiten Sensorenanordnungen (4, 5) mindestens einen Sensor für ein gasförmiges Element (Cg) umfasst,
- mindestens einen Temperatursensor (Ct) und/oder einen Feuchtesensor (Ch), die innerhalb der elektrischen Anlage (2) angeordnet sind,
- eine Verarbeitungseinheit (6), die einen Prozessor (9) umfasst, der dazu ausgestaltet ist, mindestens die folgenden Schritte auszuführen:
a1) Vergleichen der Konzentrationen des gasförmigen Elements, die mit der ersten Sensorenanordnung (4) und der zweiten Sensorenanordnung (5) erhalten wurden,
a2) Bestimmen, ausgehend von dem Vergleich des Schritts a1), ob eine Änderung der von der ersten Sensorenanordnung (4) erfassten Konzentration des gasförmigen Elements auf einem außerhalb der elektrischen Anlage (1) liegenden Faktor beruht,
b1) Bestimmen, ob eine Korrelation zwischen der von der ersten Sensorenanordnung (4) erhaltenen Konzentration des gasförmigen Elements und den Messungen des Temperatursensors (Ct) und/oder Feuchtesensors (Ch) vorliegt,
b2) Bestimmen, ausgehend vom Schritt b1), ob eine Änderung der von der ersten Sensorenanordnung (4) erfassten Konzentration des gasförmigen Elements auf einer Änderung der Temperatur oder der Feuchte innerhalb der elektrischen Anlage (1) beruht, und
c) wenn die Antwort in den Schritten a2) und b2) negativ ist, Schlussfolgern, dass die von der ersten Sensorenanordnung (4) erhaltene Konzentration des gasförmigen Elements auf einem innerhalb der elektrischen Anlage (1) liegenden Faktor beruht.

8. System nach Anspruch 7, das eine Alarmvorrichtung (11) umfasst.

9. System nach Anspruch 7 oder 8, das einen oder mehrere Aktuatoren (12) umfasst, die dazu ausgestaltet sind, die Umgebungsbedingungen innerhalb der elektrischen Anlage (1) zu regulieren.

## Claims

1. Method for monitoring for partial discharges, DP, in an electrical installation (1) comprising at least one electrical cubicle (2), the electrical cubicle (2) comprising at least one item of medium-voltage or high-voltage electrical equipment (3), the electrical installation (1) including:
- at least one first set of sensors (4) arranged inside the electrical cubicle (2) and at least one second set of sensors (5) arranged outside the electrical cubicle (2), each of the first and second sets of sensors (4, 5) comprising at least one sensor for a gaseous element (Cg);
- at least one temperature sensor (Ct) and/or humidity sensor (Ch) arranged inside the electrical installation (1),
**characterized in that** the method comprises at least the following steps:
a1) comparing the concentrations of the gaseous element that are obtained using the first set of sensors (4) and the second set of sensors (5);
a2) determining, on the basis of the comparison in step a1), whether a variation in the concentration of the gaseous element that is acquired by the first set of sensors (4) stems from a factor external to the electrical installation (1);
b1) determining whether there is a correlation between the concentration of the gaseous element that is obtained by the first set of sensors (4) and the measurements from the temperature sensor (Ct) and/or humidity sensor (Ch);
b2) determining, on the basis of the comparison in step b1), whether a variation in the concentration of the gaseous element acquired by the first set of sensors (4) stems from a variation in temperature and/or humidity inside the electrical installation (1); and
c) if the response to steps a2) and b2) is negative, deducing that the concentration of the gaseous element that is obtained by the first set of sensors (4) stems from a factor internal to the electrical installation (1).

2. Method according to Claim 1, wherein the sensor for a gaseous element of the first and second sets of sensors (4, 5) is chosen from an ozone (Os) sensor, a carbon monoxide (CO) sensor, a nitric acid (HNO₃) sensor and a nitrogen oxides (NOₓ) sensor.

3. Method according to either of the preceding claims, wherein if the response to step b2) is positive, the method further comprises a step b3) of adjusting the environmental conditions inside the electrical installation (1).

4. Method according to Claim 1 or 2, wherein if the response to step b2) is negative, the method further comprises a step c') of determining the state of wear of the material of the item of electrical equipment (3) according to the concentration of the gaseous element obtained by the first set of sensors (4).

5. Method according to any one of the preceding claims, further comprising a warning step d) if the concentration of the gaseous element that is obtained by the first set of sensors (4) is higher than a threshold value.

6. Method according to any one of the preceding claims, wherein the internal factor is wear on the item of electrical equipment (3).

7. System for monitoring for partial discharges, DP, in an electrical installation (1) comprising at least one electrical cubicle (2), the electrical cubicle (2) comprising at least one item of medium-voltage or high-voltage electrical equipment (3), the system including:
- at least one first set of sensors (4) arranged inside the electrical cubicle (2) and at least one second set of sensors (5) arranged outside the electrical cubicle (2), each of the first and second sets of sensors (4, 5) comprising at least one sensor for a gaseous element (Cg),
- at least one temperature sensor (Ct) and/or humidity sensor (Ch) arranged inside the electrical installation (2),
- a processing unit (6) comprising a processor (9) configured to implement at least the following steps:
a1) comparing the concentrations of the gaseous element obtained using the first set of sensors (4) and the second set of sensors (5);
a2) determining, on the basis of the comparison in step a1), whether a variation in the concentration of the gaseous element that is acquired by the first set of sensors (4) stems from a factor external to the electrical installation (1);
b1) determining whether there is a correlation between the concentration of the gaseous element that is obtained by the first set of sensors (4) and the measurements from the temperature sensor (Ct) and/or humidity sensor (Ch);
b2) determining, on the basis of the comparison in step b1), whether a variation in the concentration of the gaseous element acquired by the first set of sensors (4) stems from a variation in the temperature or in the humidity inside the electrical installation (1); and
c) if the response to steps a2) and b2) is negative, deducing that the concentration of the gaseous element that is obtained by the first set of sensors (4) stems from a factor internal to the electrical installation (1).

8. System according to Claim 7, comprising a warning device (11).

9. System according to Claim 7 or 8, comprising one or more actuators (12) configured to adjust the environmental conditions inside the electrical installation (1).
